# DEMANDE DE BREVET EUROPEEN

(11) **EP 1 776 965 A1**
(43) Date de publication de la demande: **25.04.2007**
(21) Numéro de dépôt: 06291597.0
(22) Date de dépôt: 12.10.2006
(51) Int. Cl.: A61L 2/28

(54) **Dispositif de test pour contrôle d'appareil de stérilisation à pévide**

(30) Priorité: 20.10.2005 FR 0510724
(71) Demandeur: MXM, 06220 Vallauris (FR)
(72) Inventeur: Martel, Paul, 83700 St-Raphael (FR); Charvin, Guy, 06600 Antibes (FR)
(74) Mandataire: Somnier, Jean-Louis

(57) **Abrégé**

Dispositif de test apte à contrôler la qualité du gaz stérilisant dans un appareil à prévide destiné à la stérilisation par ce gaz, et comprenant une feuille de mesure sur laquelle est imprimé un motif (13) en encre indicatrice sensible audit gaz de stérilisation, au moins un bloc de matériau poreux disposé d'un côté au contact de ladite feuille (4) de mesure, et un système de contention externe (6) maintenant empilé l'ensemble (12) du matériau poreux (2, 3,) et de la feuille de mesure (4); ladite feuille de mesure (4) comporte un élément de préhension (11) apte à constituer une tirette d'extraction de ladite feuille (4), le au moins dit bloc (2, 3) de matériau poreux étant immobilisée par rapport à au moins une partie du système de contention.

## Description

La présente invention a pour objet un dispositif de test pour contrôle d'appareil de stérilisation à prévide.

Le secteur technique de l'invention est le domaine de la fabrication de dispositifs de test aptes à contrôler la qualité du gaz stérilisant dans un appareil à prévide destiné à la stérilisation par ce gaz et dont on veut donc déterminer l'efficacité du vide réalisé avant l'introduction de ce gaz, puis la qualité du gaz introduit.

La présente invention est particulièrement, mais non exclusivement, destinée aux tests d'appareils à prévide et de stérilisation par la vapeur d'eau tels que les autoclaves utilisés pour la stérilisation d'équipements médicaux et d'hôpitaux.

On connaît pour cela depuis 1961 la méthode de test dite de BOWIE-DICK qui a fait l'objet de diverses publications sans qu'il soit nécessaire donc de l'expliciter dans la présente demande: l'on pourra se référer utilement à diverses demandes de brevets antérieurs qui font référence à cette méthode, telle que la demande US 4 579 715 publiée le 1er avril 1986 au nom de la Compagnie WARNER LAMBERT ou encore le brevet US 4 486 387 publié le 4 décembre 1984 au nom de la Société PROPPER MANUFACTURING Co.

On rappellera simplement ici que le but de ce test est de vérifier qu'il ne reste pas de gaz indésirable dans l'enceinte de l'appareil stérilisateur : pour cela on utilise une encre sensible au gaz de stérilisation, mais non à ces gaz indésirables et disposée suivant un motif particulier sur une feuille de mesure glissée à l'intérieur d'un pack de matériau poreux lequel est placé dans l'appareil durant un cycle test qui est effectué, jusqu'à 134°C, à une fréquence prédéterminée, avant d'utiliser cet appareil pour stériliser des équipements. Un tel test, dont le résultat est vérifié donc a posteriori à la sortie de l'appareil, permet ainsi d'être sûr qu'il n'y avait plus de gaz indésirables tel que de l'air dans l'enceinte, car ces gaz pourraient se stratifier, créer des poches dont la température ne pourrait pas être contrôlée, ou se mélanger au gaz stérilisant, nuisant alors à la qualité de la stérilisation: si le test est positif, à savoir que l'encre sensible a changé de couleur sur toute la surface du motif, on peut en déduire qu'il n'y a eu pas de gaz indésirables pendant le cycle test et qu'il en sera de même lors des phases ultérieures de stérilisation d'équipements.

Diverses demandes de brevet ont été déposées pour protéger différentes particularités de dispositifs de test dont ceux déjà cités ci-dessus et également, parmi les plus récents :
- le brevet EP 553 519 publié le 4 août 1993 au nom de la Société PROPPER MANUFACTURING Co qui décrit et revendique un empilement particulier de diverses couches de matériau poreux entourant ladite feuille de mesure, des couches de matériau imperméable et non poreux, et un emballage poreux entourant ledit empilement de couches et feuilles,
- la demande de brevet EP 628 317 publiée le 14 décembre 1994 au nom de la Société PYMAH CORPORATION qui enseigne une couche de matériau transparent au contact de la feuille de mesure pour permettre sa lecture directe quand on ressort le pack de mesure de l'autoclave
- la demande de brevet JP 2002/224198 publiée le 13 août 2002 qui décrit un dispositif électronique de contrôle du programme de stérilisation et qui s'écarte du domaine technique de la présente invention basée sur la lecture d'une feuille de mesure à encre indicatrice sensible au gaz de stérilisation.

Le problème posé et non résolu par l'ensemble des documents de l'art antérieur, sauf par la demande de brevet EP 628 317 citée ci-dessus et analysée ci-après mais avec un dispositif différent, est de simplifier la manipulation du pack de test en sortie de l'autoclave pour lire la feuille de mesure. En effet à ce jour il est nécessaire d'ouvrir complètement l'emballage, de séparer les divers matériaux qui entourent la feuille de mesure, d'extraire celle-ci pour contrôler le changement de couleur de l'encre sensible et ensuite la stocker pour garder la traçabilité du test effectué, l'ensemble des autres matériaux constituant le pack de test étant jeté. Certes la demande de brevet EP 628 317 évoquée précédemment permet une lecture directe au travers des matériaux transparents sans avoir à ouvrir le pack mais complique alors le stockage de ces résultats de tests puisse que ce sont les packs entiers qu'il faut garder, à moins de les ouvrir comme pour les précédents avec donc les mêmes inconvénients.

Une solution au problème posé est un dispositif ou pack de test, tel que ceux cités ci-dessus, c'est-à-dire apte à contrôler la qualité du gaz stérilisant dans un appareil à prévide destiné à la stérilisation par ce gaz et comprenant :
- une feuille de mesure sur laquelle est imprimée un motif en encre indicatrice sensible au dit gaz de stérilisation,
- et un pack constitué d'au moins un bloc de matériau poreux, pouvant être constitué par une pile dudit matériau, disposé d'un côté au contact de ladite feuille de mesure et apte à laisser pénétrer et diffuser les gaz présents dans l'enceinte de l'appareil vers ou depuis la surface encrée de la feuille de mesure, et d'un système de contention externe, pouvant être un emballage enveloppant, maintenant empilé l'ensemble du matériau poreux et de la feuille de mesure tout en permettant le passage du gaz de stérilisation et des gaz indésirables dans et ou hors du volume intérieur du dit ensemble.
   Selon l'invention ladite feuille de mesure comporte un élément de préhension apte à constituer une tirette d'extraction de ladite feuille de mesure hors de l'ensemble ci-dessus et qui déborde au moins sur un côté de celui-ci, à l'extérieur du contour au moins de la partie du matériau poreux disposé au moins d'un côté de ladite feuille de mesure, adjacente à celle-ci, le au moins dit bloc de matériau poreux étant immobilisé par rapport à au moins une partie dudit système de contention et préservant l'intégrité du pack du dispositif de test, même quand on tire sur l'élément de préhension pour extraire la feuille de mesure, lequel dispositif de test étant apte à répondre aux conditions de la méthode de test dite de BOWIE-DICK pour les appareils de stérilisation dans lesquels le gaz de stérilisation est la vapeur d'eau.
   Le résultat est un nouveau type de dispositifs de test répondant au problème posé et aux divers inconvénients des dispositifs actuels puisque on peut ainsi grâce d'une part à l'élément de préhension qui doit dépassé suffisamment du bloc poreux pour le saisir aisément, et d'autre part à l'architecture du pack, retirer facilement la feuille de mesure du pack de test, sans avoir à ouvrir complètement et préalablement le système de contention qui le maintient, puis à écarter l'ensemble des matériaux qui l'entourent, ceux-ci restant au contraire dans le système de contention ou emballage. Cette disposition de caractéristiques selon l'invention simplifie donc la manipulation et préserve ainsi l'intégrité du pack du dispositif de test, qui n'est donc pas ouvert et peut être ensuite jeter tel que, au lieu d'avoir à le faire pour les multiples éléments séparés les uns des autres comme dans les packs actuels.
   Ledit élément de préhension peut être constitué soit par une partie de la feuille de mesure elle-même soit par un élément rajouté à celle-ci mais devant être suffisamment solidaire de la feuille de mesure pour pouvoir tirer sur celle-ci sans risque de rupture afin de l'extraire du pack de mesure.
   On pourrait citer d'autres avantages de la présente invention mais ceux cités ci-dessus en montrent déjà suffisamment pour en prouver la nouveauté et l'intérêt. La description ci-après et les figures ci-jointes représentent des exemples de réalisation de l'invention mais n'ont aucun caractère limitatif: d'autres réalisations sont possibles dans le cadre de la portée de l'étendue de cette invention.
- La figure 1 est une vue perspective de l'ensemble des éléments constituant l'intérieur du dispositif suivant l'invention avant qu'ils soient empilés les uns contre les autres et placés dans un système de contention qui les maintient ensemble et qui sera décrit et nommé ici en tant qu'emballage, constituant alors un pack de test.
- La figure 2 est une vue perspective d'un dispositif de test suivant l'invention en position ouverte après le test et suivant un premier mode de réalisation.
- Les figures 3A et 3B sont des vues de dessus d'un ensemble d'éléments constituant l'intérieur d'un dispositif de test suivant l'invention, tel que représenté sur la figure 1, suivant deux modes de réalisation de l'élément de préhension de la feuille de mesure.
- La figure 4 est une vue perspective d'un dispositif de test complet tel que représenté sur la figure 2 mais en position fermée, prêt à être utilisé ou juste après son utilisation et avant ouverture pour accéder à l'élément de préhension de la feuille de mesure.
- La figure 5 est une vue perspective d'un autre mode de réalisation d'un dispositif de test suivant l'invention en position de montage.
- La figure 6 est une vue perspective d'un autre mode de réalisation d'un dispositif de test suivant l'invention également pendant son montage.

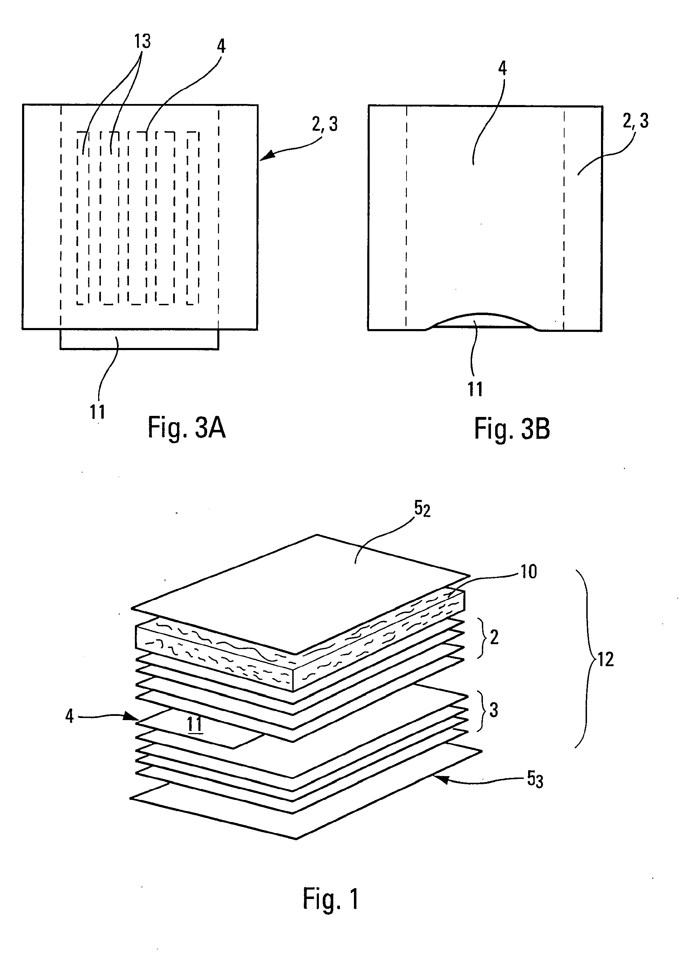

Selon la figure 1 le dispositif de test suivant l'invention comporte au moins deux blocs 2, 3 de matériau poreux, qui sont suivant les présents modes de réalisation décrits, des piles de feuilles poreuses disposées de part et d'autre de ladite feuille 4 de mesure et au moins deux feuilles imperméables 5₂, 5₃ au gaz de stérilisation et aux gaz indésirables prenant en sandwich lesdites piles 2, 3.

L'ensemble 12 de ces blocs et feuilles ainsi disposées est prêt à être resserré pour être mis à l'intérieur de l'emballage 6 tel que représenté sur les figures 2, 4, 5 ou 6 : il doit ensuite rester compact, de telle sorte qu'aucun élément ne puisse bouger par rapport à la feuille de mesure 4.

Pour aider au maintien des différents blocs et/ou feuilles qui doivent rester pressées d'une façon uniforme les unes contre les autres afin d'avoir une homogénéité de pénétration ou d'évacuation de gaz, respectivement dans ou vers l'extérieur du dit ensemble 12, le dispositif de test comporte d'une manière connue une pièce parallélépipédique en matériau souple 10 compressible apte à maintenir ledit ensemble 12 de feuilles 2, 3, 4 et 5 bien empilées les unes contre les autres à l'intérieur de l'emballage externe 6 : ladite pièce en matériau souple 10 est disposée entre ledit emballage externe 6, ou une des feuilles 5₂, 5₃ imperméables, et l'une des deux piles 2, 3 de feuilles poreuses disposées contre un des côtés de ladite feuille 4 de mesure.

Selon le mode de réalisation de la figure 2 ainsi que suivant les vues de dessus des figures 3, ledit élément de préhension 11 de ladite feuille de mesure 4 déborde au moins sur un côté du dit ensemble 12 et forme une languette 11, qui est rabattue en position de test perpendiculairement au plan de la feuille de mesure 4 est apte à être remis dans le plan de celle-ci pour pouvoir facilement extraire cette dernière de l'ensemble 12 hors du pack 1 constituant le dispositif de test, une fois la face 7 ouvrante de l'emballage 6 dégagée comme représenté sur la figure 2.

Dans le mode de réalisation suivant la figure 3B ledit élément de préhension 11 de ladite feuille de mesure 4 est obtenu par une simple échancrure réalisée dans au moins une partie de matériau poreux 2, 3 pouvant être constitué de piles de feuilles, disposé au moins d'un côté de ladite feuille de mesure 4.

Pour faciliter son extraction et éviter le frottement éventuel de la feuille de mesure 4 contre les parois latérales de l'emballage, la largeur de la feuille de mesure 4, considérée parallèlement à son côté comportant son élément de préhension 11, est plus étroite que celle du matériau poreux 2,3 disposé au moins d'un côté au contact de la feuille de mesure.

Dans un mode préférentiel de réalisation le système de contention extérieur 6 est une boîte formant emballage en matériau semi rigide fermée sur au moins deux faces latérales opposées, dont au moins une partie de la surface de l'une 7 est apte à être ouverte face à l'élément de préhension 11 de ladite feuille de mesure 4 et à laisser une ouverture au moins égale à la largeur de celle-ci, permettant ainsi d'accéder directement à cet élément de préhension et d'extraire par traction la feuille de mesure 4 hors du pack par cette face 7 ouvrante de l'emballage faisant face à l'élément de préhension 11.

Dans le mode de réalisation de la figure 2 la face 7 ouvrante du système de contention ou emballage 6 comporte au moins deux rabats latéraux 7₂ solidaires des angles communs avec les deux autres faces latérales de l'emballage, lesquels rabats latéraux laissent en position fermée un espace entre eux au moins égal à la largeur de la feuille de mesure 4 et sont aptes ainsi à immobiliser le ou les matériaux poreux 2,3 qui viennent se bloquer contre ces rabats 7₂, maintenus eux-mêmes en position fermée par les rabats longitudinaux 7₁ qui les recouvrent et qui sont simplement dégagés pour accéder à l'élément de préhension 11 sans être cependant complètement ouverts.

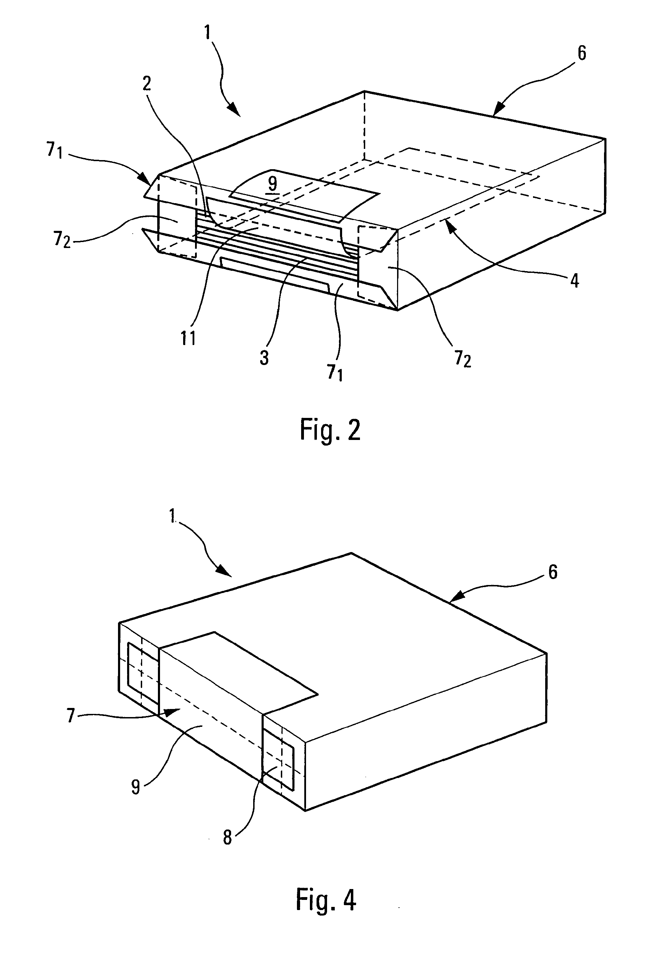

Pour cela et afin de faciliter sa prise en main, l'élément de préhension 11 est de préférence rabattu à l'extérieur d'un des rabats longitudinaux 7₁ et est recouvert par une partie mobile découvrable 8 obturant et maintenant ladite face 7 en position fermée, et apte à être manipulée par arrachement ou simplement pivotement pour permettre l'ouverture de celle-ci, comme représenté sur la figure 4 ainsi que sur la figure 2 après avoir été manipulée, en fait ici dans l'exemple de réalisation représenté par arrachement complet.

Pour maintenir en place ladite partie mobile découvrable 8 le dispositif de test suivant l'invention comporte une étiquette 9 collée au moins sur cette face 7 et dont une partie au moins et apte à être enlevée pour libérer la partie découvrable 8, comme représenté sur la figure 4.

Dans un autre mode de réalisation le système de contention extérieur 6 peut être une boîte formant emballage en matière semi rigide fermée sur au moins deux faces latérales opposées dont une 7 est ouvrante face à l'élément de préhension 11 de ladite feuille de mesure 4, permettant d'y accéder et de l'extraire, et dans lequel soit l'unique bloc de matériau poreux, soit les deux blocs 2, 3 qui entourent ladite feuille de mesure 4, sont collées au moins contre une face, différente de celle 7 ouvrante de l'emballage 6.

Ledit système de contention peut également être composé de seulement quatre faces entourant ledit ensemble 12 comme un fourreau dont deux faces latérales opposées, dont celle ouvrante 7, et deux faces supérieures et inférieures parallèles à la feuille de mesure 4 : les deux autres faces latérales ne sont pas fermées dans la mesure bien sûr où l'ensemble 12 est bien immobilisé à l'intérieur du fourreau 6 formant le système de contention; celui doit être suffisamment rigide pour maintenir l'intégrité du pack, et l'ensemble 12 des éléments empilés à l'intérieur, sauf la feuille de mesure 4, étant maintenus solidaires, tel que justement par collage sur la face latérale opposée à celle ouvrante 7.

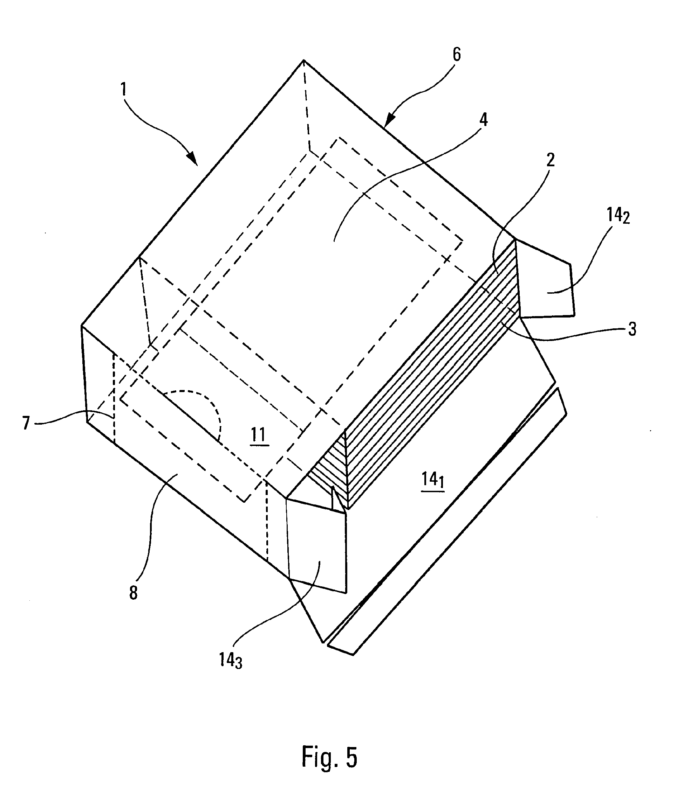

Dans un autre mode de réalisation tel que représenté sur la figure 5, la face 7, du côté de l'ouverture de l'emballage 6 pour accéder à l'élément de préhension 11, comporte une bande détachable 8 de largeur au moins égale à celle de la feuille de mesure 4 et pouvant être la totalité de la face 7, mais ne comporte pas de rabat; c'est au moins une autre face latérale adjacente 14 qui en comprend et qui s'ouvre pour y glisser l'ensemble 12 des éléments empilés du dispositif de test. L'un de ces rabats 14₃ situé du côté de l'élément de préhension 11, qui est simplement laissé dans le plan de la feuille de mesure 4, est replié sur lui-même pour une fois fermé permettre le blocage de l'ensemble 12 dans l'emballage 6. Pour des raisons de symétrie et d'équilibre de blocage, l'autre face opposée à celle 14 représentée présente les mêmes caractéristiques.

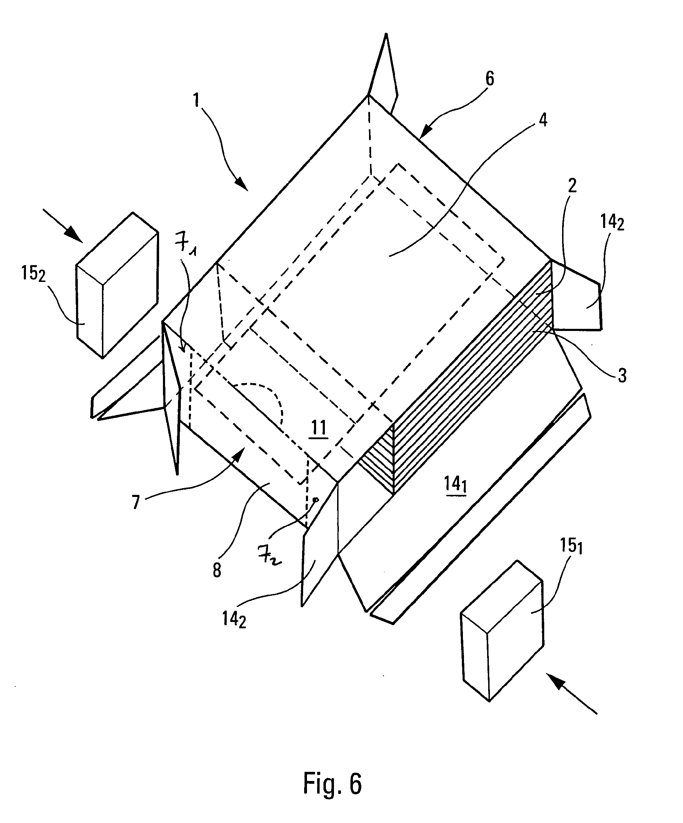

Dans une variante au mode de réalisation ci-dessus de la figure 5 et tel que représenté sur la figure 6, le blocage de l'ensemble 12 est réalisé, non plus par des rabats de l'emballage 6, mais par l'adjonction de deux petits blocs de mousse 15, ou de tout autre matériau, supportant le cycle de stérilisation : ces blocs 15 sont glissés de part et d'autre de l'élément de préhension 11 de la feuille de mesure 4 entre la face ouvrante 7 et l'ensemble 12 pour maintenir celui-ci immobilisé à l'intérieur du pack, afin d'une part d'éviter tout mouvement lors de son transport et de son utilisation, puis d'autre part de maintenir (par appui sur les côtés 7₁ et 7₂ de la face 7 non totalement ouvrante et restant solidaires de l'emballage, une fois la bande détachable 8 de largeur au moins égale à celle de la feuille de mesure 4 enlevée) cet ensemble 12 par rapport à la face 7 quand, une fois la bande détachable 8 enlevée, on extrait la feuille de mesure 4 sans que les autres éléments qui constituent l'ensemble 12 ne soient entraînés en même temps.

On peut alors ainsi rapidement contrôler sans avoir à défaire tout le pack, comment a réagi l'encre chimique réactive déposée sur la feuille de mesure 4 et constituant un motif 13.

Dans d'autres modes de réalisation tels que suivant la figure 3B, les rabats doubles 14₃ ou les blocs de mousse 15 ne sont bien sûr pas nécessaires.

Suivant les modes de réalisation décrits ci-dessus, l'extraction de la feuille de mesure 4 peut se faire en gardant le pack en position « à plat » soit avec la feuille de mesure 4 horizontale, ce qui est la position la plus pratique ; cependant dans un autre mode de réalisation suivant lequel il n'y aurait pas de blocage des blocs ou piles 2, 3 de matériau poreux, soit par appui contre un ou des éléments du système de contention ou emballage, soit par collage contre au moins une face de celui-ci, l'immobilisation de ces blocs ou piles peut être obtenue en maintenant le pack en position verticale, l'extraction de la feuille de mesure 4 étant réalisée en tirant son élément de préhension 11 vers le haut, ce qui n'est certes pas une position très pratique, les blocs ou piles 2, 3 de matériau poreux restant dans le système de contention ou emballage, par leur propre poids et leur frottement contre les parois intérieures de celui-ci.

## Revendications

1. Dispositif de test (1) apte à contrôler la qualité du gaz stérilisant dans un appareil à prévide destiné à la stérilisation par ce gaz, et comprenant une feuille (4) de mesure sur laquelle est imprimé un motif (13) en encre indicatrice sensible audit gaz de stérilisation, et un pack constitué d'au moins un bloc (2, 3) de matériau poreux disposé d'un côté au contact de ladite feuille (4) de mesure, et apte à laisser pénétrer et diffuser les gaz présents dans l'enceinte de l'appareil vers ou depuis la surface encrée de la feuille de mesure (4) et d'un système de contention externe (6) maintenant empilé l'ensemble (12) du matériau poreux (2, 3,) et de la feuille de mesure (4) tout en permettant le passage du gaz de stérilisation et des gaz indésirables, dans et/ou hors du volume intérieur dudit ensemble (12), **caractérisé en ce que** ladite feuille de mesure (4) comporte un élément de préhension (11) apte à constituer une tirette d'extraction de ladite feuille (4) de mesure hors de l'ensemble (12) et qui déborde au moins sur un côté de celui-ci, à l'extérieur du contour au moins de la partie du matériau poreux (2, 3), disposé au moins d'un côté de ladite feuille de mesure (4), adjacente à celle-ci, le au moins dit bloc (2, 3) de matériau poreux étant immobilisée par rapport à au moins une partie dudit système de contention (6), et préservant l'intégrité du pack du dispositif de test (1) même quand on tire sur l'élément de préhension (11) pour extraire la feuille de mesure (4), lequel dispositif étant apte à répondre aux conditions de la méthode de test dite de BOWIE-DICK pour des appareils de stérilisation dans lesquels le gaz de stérilisation est la vapeur d'eau.

2. Dispositif de test selon la revendication 1 **caractérisé en ce que** ledit élément de préhension (11) est constitué par une partie de la feuille de mesure (4) elle-même.

3. Dispositif de test selon l'une quelconque des revendications 1 à 2, **caractérisé en ce que** ledit élément de préhension (11) de ladite feuille de mesure (4), débordant au moins sur un côté dudit ensemble (12), forme une languette (11) rabattue en position de test perpendiculairement au plan de la feuille de mesure (4), et apte à être remis dans le plan de celle-ci.

4. Dispositif de test selon l'une quelconque des revendications 1 à 3, **caractérisé en ce que** la largeur de la feuille de mesure (4), considérée parallèlement à son côté comportant son élément de préhension (11), est plus étroite que celle du matériau poreux (2, 3) disposé au moins d'un côté au contact de la feuille de mesure.

5. Dispositif de test selon la revendication 4, **caractérisé en ce que** le système de contention extérieur (6) est une boîte formant emballage en matériau semi rigide, fermée sur au moins deux faces latérales opposés dont au moins une partie de la surface de l'une 7 est apte à être ouverte face à l'élément de préhension (11) de ladite feuille de mesure (4) et à laisser une ouverture au moins égale à la largeur de celle-ci.

6. Dispositif de test selon la revendication 5, **caractérisé en ce que** la face (7) ouvrante du système de contention (6) comporte au moins deux rabats latéraux (7₂), solidaires des angles communs avec les deux autres faces latérales de l'emballage, qui laissent en position fermée un espace entre eux au moins égal à la largeur de la feuille de mesure (4) et sont aptes à immobiliser le matériau poreux (2, 3).

7. Dispositif de test selon la revendication 6, **caractérisé en ce que** le système de contention extérieur (6) est une boîte formant emballage en matériau semi rigide, fermée sur au moins deux faces latérales opposées, dont une (7) est ouvrante face à l'élément de préhension (11) de ladite feuille de mesure (4), et dans lequel le au moins bloc (2, 3) de matériau poreux est collée au moins contre une face, différente de celle (7) ouvrante, de l'emballage (6).

8. Dispositif de test selon l'une quelconque des revendications 5 à 7, **caractérisé en ce que** la face (7) ouvrante du système de contention (6) comporte une partie mobile découvrable (8) obturant et maintenant ladite face (7) en position fermée, et apte à être manipulée pour permettre l'ouverture de celle-ci.

9. Dispositif de test selon la revendication 8, **caractérisé en ce qu'**il comporte une étiquette (9) collée au moins sur la face (7) et dont une partie au moins est apte à être enlevée pour libérer la partie découvrable (8).

10. Dispositif de test selon l'une quelconque des revendications 1 à 9, **caractérisé en ce qu'**il comporte au moins deux piles (2, 3) de feuilles poreuses disposées de part et d'autre de ladite feuille (4) de mesure et au moins deux feuilles imperméables (5) au gaz de stérilisation et aux gaz indésirables prenant en sandwich lesdites piles (2, 3) à l'intérieur de l'emballage (6) et faisant partie de l'ensemble (12).
